(19) **Europäisches Patentamt / European Patent Office / Office européen des brevets**

(11) **EP 4 620 938 A1**

(12) **EUROPEAN PATENT APPLICATION**
published in accordance with Art. 153(4) EPC

(43) Date of publication:
24.09.2025 Bulletin 2025/39

(21) Application number: 23891520.1

(22) Date of filing: 13.11.2023

(51) International Patent Classification (IPC):
C07C 7/12 (2006.01)  C07C 9/04 (2006.01)
C07C 9/06 (2006.01)  C07C 9/08 (2006.01)
C07C 9/10 (2006.01)  C07C 9/12 (2006.01)
C07C 11/04 (2006.01)  B01D 39/06 (2006.01)
B01D 39/16 (2006.01)  B01D 39/20 (2006.01)
B01D 53/04 (2006.01)  B01D 53/22 (2006.01)

(52) Cooperative Patent Classification (CPC):
C07C 7/12; C07C 9/04; C07C 9/06; C07C 9/08;
C07C 9/10; C07C 9/12; C07C 11/04; B01D 39/06;
B01D 39/16; B01D 39/20; B01D 53/04; B01D 53/22

(86) International application number:
PCT/JP2023/040727

(87) International publication number:
WO 2024/106373 (23.05.2024 Gazette 2024/21)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 16.11.2022 JP 2022183261

(71) Applicant: **Resonac Corporation**
Tokyo 105-7325 (JP)

(72) Inventors:
• **MATSUI, Kazuma**
Tokyo 105-7325 (JP)
• **OHTSUKI, Kazuaki**
Tokyo 105-7325 (JP)

(74) Representative: **Strehl & Partner mbB**
**Maximilianstrasse 54**
**80538 München (DE)**

(54) **METHOD FOR PRODUCING HYDROCARBON AND METHOD FOR PRODUCING SILICON CARBIDE**

(57)     Provided is a method for producing hydrocarbon in which high-purity hydrocarbon having a low content of hydrogen gas, nitrogen gas, oxygen gas, boron, aluminum, phosphorus, sulfur, titanium, vanadium, chromium, and molybdenum can be produced. The method for producing hydrocarbon includes: a hydrogen gas removal step of removing hydrogen gas from crude hydrocarbon containing hydrocarbon having 1 or more and 4 or less carbon atoms and the hydrogen gas to obtain low-hydrogen hydrocarbon in which a content of hydrogen gas is 100 ppm by volume or less; and an adsorption step of bringing the low-hydrogen hydrocarbon into contact with an adsorbent to obtain high-purity hydrocarbon in which a content of hydrogen gas is 80 ppm by volume or less, a total content of nitrogen gas and oxygen gas is 5 ppm by volume or less, and a total content of boron, aluminum, phosphorus, sulfur, titanium, vanadium, chromium, and molybdenum is 250 ppb by mass or less. The adsorbent includes a crystal having pores with a pore diameter of more than 0.3 nm and 3.5 nm or less, and a crystal form of the crystal is not a mordenite type.

FIG. 5

**Description**

Technical Field

The present invention relates to a method for producing hydrocarbon and a method for producing silicon carbide.

Background Art

Single-crystal silicon carbide (SiC) has excellent electrical characteristics in that the bandgap is about 3 times and the breakdown field strength is 10 times as compared to silicon (Si), and thus is useful as a material of a power semiconductor. As a raw material for producing single-crystal silicon carbide, high-purity hydrocarbon is used. For example, PTL 1 discloses a purification method of propane in which impurities are removed from low-purity propane ($C_3H_8$) to increase the purity. According to the technique disclosed in PTL 1, high-purity propane having a purity of 99.99% by volume or more can be obtained, and the high-purity propane is useful as a raw material of the single-crystal silicon carbide.

As the impurities to be removed from the low-purity propane, PTL 1 discloses ethane ($C_2H_6$), propylene ($C_3H_6$), n-butane ($C_4H_{10}$), isobutane ($C_4H_{10}$), water ($H_2O$), nitrogen gas ($N_2$), oxygen gas ($O_2$), and carbon dioxide ($CO_2$). However, the low-purity propane may contain impurities other than the above-described examples.

Citation List

Patent Literatures

PTL 1: JP 5822299 B
PTL 2: WO 2016/121622

Summary of Invention

Technical Problem

For example, hydrogen gas ($H_2$) is used as a raw material for synthesizing propane from propylene. Therefore, there is a concern that the obtained propane may contain a large amount of hydrogen gas (refer to PTL 2). When propane contains hydrogen gas, there is a concern that it is difficult to remove nitrogen gas or oxygen gas from the propane.

In addition, when single-crystal silicon carbide contains an element such as nitrogen (N), oxygen (O), boron (B), aluminum (Al), phosphorus (P), sulfur (S), titanium (Ti), vanadium (V), chromium (Cr), or molybdenum (Mo), there is a concern that the element may function a dopant or an oxide of the element may oxidize the silicon carbide. As a result, electrical characteristics of the silicon carbide change, and thus there is a concern that this change may have an adverse effect on the quality of a semiconductor.

Accordingly, it is desirable that hydrocarbon that is used as a raw material for producing single-crystal silicon carbide has low contents of hydrogen gas, nitrogen gas, oxygen gas, boron, aluminum, phosphorus, sulfur, titanium, vanadium, chromium, and molybdenum.

An object of the present invention is to provide a method for producing hydrocarbon in which high-purity hydrocarbon having low contents of hydrogen gas, nitrogen gas, oxygen gas, boron, aluminum, phosphorus, sulfur, titanium, vanadium, chromium, and molybdenum can be produced, and a method for producing silicon carbide in which silicon carbide having excellent electrical characteristics can be produced.

Solution to Problem

To solve the above objects, one aspect of the present invention is as the following [1] to [8].

[1] A method for producing hydrocarbon, the method including:

a hydrogen gas removal step of removing hydrogen gas from crude hydrocarbon containing hydrocarbon having 1 or more and 4 or less carbon atoms and the hydrogen gas to obtain low-hydrogen hydrocarbon in which a content of hydrogen gas is 100 ppm by volume or less; and
an adsorption step of bringing the low-hydrogen hydrocarbon into contact with an adsorbent to obtain high-purity hydrocarbon in which a content of hydrogen gas is 80 ppm by volume or less, a total content of nitrogen gas and oxygen gas is 5 ppm by volume or less, and a total content of boron, aluminum, phosphorus, sulfur, titanium, vanadium, chromium, and molybdenum is 250 ppb by mass or less,

in which the adsorbent includes a crystal having pores with a pore diameter of more than 0.3 nm and 3.5 nm or less, and a crystal form of the crystal is not a mordenite type.

[2] The method for producing hydrocarbon according to [1], in which the hydrocarbon is at least one among methane, ethane, ethylene, propane, n-butane, and isobutane.

[3] The method for producing hydrocarbon according to [1], in which the hydrocarbon is at least one among propane and ethylene.

[4] The method for producing hydrocarbon according to [1], in which the adsorption step is a step of adsorbing at least one among the nitrogen gas and the oxygen gas on the adsorbent from the low-hydrogen hydrocarbon.

[5] The method for producing hydrocarbon according to [1], in which the adsorption step is a step of adsorbing at least one among boron, aluminum, phosphorus, sulfur, titanium, vanadium, chromium, and molybdenum on the adsorbent from the low-hydrogen hydrocarbon.

[6] The method for producing hydrocarbon according to [1], in which the hydrogen gas removal step is a step of removing the hydrogen gas from the crude hydrocarbon using a separation membrane.

[7] The method for producing hydrocarbon according to [1], further including a filtration step of causing the crude hydrocarbon, the low-hydrogen hydrocarbon, or the high-purity hydrocarbon to pass through a filter medium to remove at least one among boron, aluminum, phosphorus, sulfur, titanium, vanadium, chromium, and molybdenum from the crude hydrocarbon, the low-hydrogen hydrocarbon, and the high-purity hydrocarbon,

in which the filter medium is at least one filter among a membrane filter, a sintered metal filter, and a filter including a plurality of metal halide pellets.

[8] A method for producing silicon carbide in which silicon carbide is produced by using, as a raw material, high-purity hydrocarbon produced with the method for producing hydrocarbon according to any one of [1] to [7] .

Advantageous Effects of Invention

With the method for producing hydrocarbon according to the present invention, high-purity hydrocarbon having low contents of hydrogen gas, nitrogen gas, oxygen gas, boron, aluminum, phosphorus, sulfur, titanium, vanadium, chromium, and molybdenum can be produced. In addition, with the method for producing silicon carbide according to the present invention, silicon carbide having excellent electrical characteristics can be produced.

Brief Description of Drawings

FIG. 1 is a schematic view illustrating an example of a membrane separation device used in a hydrogen gas removal step;

FIG. 2 is a schematic view illustrating an example of an adsorption device used in an adsorption step;

FIG. 3 is a schematic view illustrating an example of a filtration device used in a filtration step;

FIG. 4 is a schematic view illustrating an example of a preparation device for preparing an analysis sample; and

FIG. 5 is a schematic view illustrating an example of a device for producing hydrocarbon that continuously performs the hydrogen gas removal step, the adsorption step, and the filtration step.

Description of Embodiments

Embodiments of the present invention will now be described. The embodiments are merely examples of the present invention, and the present invention is not limited to the embodiments. Various modifications or improvements can be made in the embodiments, and such modifications and improvements can be encompassed by the present invention.

A method for producing hydrocarbon according to the present embodiment includes: a hydrogen gas removal step of removing hydrogen gas from crude hydrocarbon containing hydrocarbon having 1 or more and 4 or less carbon atoms and the hydrogen gas to obtain low-hydrogen hydrocarbon in which a content of hydrogen gas is 100 ppm by volume or less; and an adsorption step of bringing the low-hydrogen hydrocarbon into contact with an adsorbent to obtain high-purity hydrocarbon in which a content of hydrogen gas is 80 ppm by volume or less, a total content of nitrogen gas and oxygen gas is 5 ppm by volume or less, and a total content of boron, aluminum, phosphorus, sulfur, titanium, vanadium, chromium, and molybdenum is 250 ppb by mass or less.

The adsorbent includes a crystal having pores with a pore diameter of more than 0.3 nm and 3.5 nm or less, and a crystal form of the crystal is not a mordenite type.

With the method for producing hydrocarbon according to the present embodiment, high-purity hydrocarbon having low contents of hydrogen gas, nitrogen gas, oxygen gas, boron, aluminum, phosphorus, sulfur, titanium, vanadium, chromium, and molybdenum can be produced. The details will be described below.

The hydrocarbon having 1 or more and 4 or less carbon atoms may contain hydrogen gas, nitrogen gas, and oxygen gas as impurities. When hydrocarbon contains hydrogen gas, nitrogen gas and oxygen gas are not likely to be removed from the hydrocarbon. Therefore, when a treatment of removing nitrogen gas and oxygen gas is performed after reducing the content of hydrogen gas, nitrogen gas and oxygen gas are likely to be removed from the hydrocarbon.

In the method for producing hydrocarbon according to the present embodiment, the adsorption step is performed after performing the hydrogen gas removal step of removing hydrogen gas from crude hydrocarbon to obtain low-hydrogen hydrocarbon. Therefore, the nitrogen gas and the oxygen gas in the hydrocarbon are effectively adsorbed by the adsorbent, and high-purity hydrocarbon can be obtained.

The obtained high-purity hydrocarbon has low contents of hydrogen gas, nitrogen gas, oxygen gas, boron, aluminum, phosphorus, sulfur, titanium, vanadium, chromium, and molybdenum. Therefore, the high-purity hydrocarbon is suitable as a raw material for producing silicon carbide and is particularly suitable as a raw material for producing single-crystal silicon carbide that is useful as a material of a power semiconductor.

In addition, the obtained high-purity hydrocarbon has a low content of oxygen gas, and thus combustion or explosion is not likely to occur. Further, the obtained high-purity hydrocarbon has a low content of hydrogen gas, and thus hydrogen embrittlement of metal is not likely to occur. For example, hydrogen embrittlement is not likely to occur in metal for forming a container that stores the high-purity hydrocarbon or in metal for forming a pipe through which the high-purity hydrocarbon is blown, and thus the storage container or the pipe is not likely to deteriorate.

In addition, a method for producing silicon carbide according to the present embodiment is a method for producing silicon carbide by using, as a raw material, high-purity hydrocarbon produced with the method for producing hydrocarbon according to the present embodiment. The high-purity hydrocarbon has low contents of various impurities as described above. Therefore, when silicon carbide is produced by using, as a raw material, high-purity hydrocarbon produced with the method for producing hydrocarbon according to the present embodiment, silicon carbide having low contents of the above-described elements (hydrogen, nitrogen, oxygen, boron, aluminum, phosphorus, sulfur, titanium, vanadium, chromium, and molybdenum) can be produced. Accordingly, silicon carbide produced with the method for producing silicon carbide according to the present embodiment has excellent electrical characteristics, and thus by using the silicon carbide, a semiconductor having excellent characteristics can be produced.

[Kind of Hydrocarbon]

The kind of hydrocarbon is not particularly limited as long as it has 1 or more and 4 or less carbon atoms. However, from the viewpoints of a high vapor pressure and easy supply, alkane having 1 or more and 4 or less carbon atoms or alkene or alkyne having 2 or more and 4 or less carbon atoms is preferable.

Examples of the alkane include methane, ethane, propane, n-butane, and isobutane. Examples of the alkene include ethylene, propylene, 1-butene, 2-butene (including a cis form and a trans form), and isobutene. Examples of the alkyne include acetylene, propyne, 1-butyne, and 2-butyne.

These hydrocarbons may be used alone or as a mixture of two or more kinds thereof. In addition, among these hydrocarbons, methane, ethane, propane, n-butane, isobutane, or ethylene is preferable from the viewpoint of easy availability.

[Impurities]

Examples of the impurities in the hydrocarbon include a compound (excluding hydrocarbon having 1 or more and 4 or less carbon atoms) or an elemental substance including elements in the first period to the fifth period of the periodic table. In particular, gaseous impurities such as hydrogen gas, nitrogen gas, or oxygen gas or solid impurities such as a compound or an elemental substance including at least one among boron, aluminum, phosphorus, sulfur, titanium, vanadium, chromium, and molybdenum in the molecule are likely to be mixed in hydrocarbon. Examples of the compound having these elements in the molecule include an oxide, a nitride, a halide, a hydride, and a hydroxide. These compounds may be present in the form of a particle, a cluster, a complex, or the like.

When the hydrocarbon contains hydrogen gas among these impurities, the storage container or the pipe of hydrocarbon may deteriorate. In addition, when hydrocarbon contains these impurities, in a case where high purity is required for hydrocarbon, for example, in a film forming step of silicon carbide or a diamond wafer during production of a semiconductor using hydrocarbon, characteristics of the product may deteriorate. Therefore, it is desirable to remove the impurities as much as possible from hydrocarbon by purification. On the other hand, excessive purification leads to an increase in the production cost of hydrocarbon. Therefore, a small amount of impurities may be contained. The concentration of impurities in hydrocarbon can be determined using a method such as gas chromatography or inductively coupled plasma mass spectrometry (ICP-MS).

[Crude Hydrocarbon]

Crude hydrocarbon contains hydrocarbon having 1 or more and 4 or less carbon atoms and hydrogen gas. The crude hydrocarbon may contain the above-described impurities other than hydrogen gas. Examples of the crude hydrocarbon include crude propane that is synthesized by causing hydrogen gas to react with propylene, and there is a concern that this crude propane may contain a large amount of hydrogen gas.

In addition, when the content of hydrogen gas in the crude hydrocarbon is more than 100 ppm by volume, the hydrogen gas removal step is performed on the crude hydrocarbon to adjust the content of hydrogen gas to be 100 ppm by volume or less.

When the content of hydrogen gas in the crude hydrocarbon is more than 100 ppm by volume, the content of hydrogen gas needs to be adjusted to be 100 ppm by volume or less by performing the hydrogen gas removal step. However, when the content of hydrogen gas is 100 ppm by volume or less, the hydrogen gas removal step does not need to be performed. That is, when the content of hydrogen gas is 100 ppm by volume or less, it can be said that the crude hydrocarbon is low-hydrogen hydrocarbon. Therefore, the hydrogen gas removal step may be skipped to perform the adsorption step on the crude hydrocarbon.

[Low-Hydrogen Hydrocarbon]

The low-hydrogen hydrocarbon is hydrocarbon in which the content of hydrogen gas is 100 ppm by volume or less. By performing the hydrogen gas removal step on the crude hydrocarbon to remove hydrogen gas from the crude hydrocarbon, low-hydrogen hydrocarbon can be obtained.

[High-Purity Hydrocarbon]

The high-purity hydrocarbon is hydrocarbon in which a content of hydrogen gas is 80 ppm by volume or less, a total content of nitrogen gas and oxygen gas is 5 ppm by volume or less, and a total content of boron, aluminum, phosphorus, sulfur, titanium, vanadium, chromium, and molybdenum is 250 ppb by mass or less. By bringing the low-hydrogen hydrocarbon into contact with the adsorbent to perform the adsorption step, impurities can be removed from the low-hydrogen hydrocarbon to obtain high-purity hydrocarbon.

The content of hydrogen gas in the high-purity hydrocarbon needs to be 80 ppm by volume or less and is preferably 5 ppm by volume or more and 80 ppm by volume or less and more preferably 40 ppm by volume or more and 74 ppm by volume or less.

In addition, the total content of nitrogen gas and oxygen gas in the high-purity hydrocarbon needs to be 5 ppm by volume or less and is preferably 0.2 ppm by volume or more and 3 ppm by volume or less, more preferably 0.3 ppm by volume or more and 2 ppm by volume or less, and still more preferably 0.4 ppm by volume or more and 1 ppm by volume or less.

Further, the total content of boron, aluminum, phosphorus, sulfur, titanium, vanadium, chromium, and molybdenum in the high-purity hydrocarbon needs to be 250 ppb by mass or less and is preferably 1 ppb by mass or more and 100 ppb by mass or less, more preferably 3 ppb by mass or more and 50 ppb by mass or less, and still more preferably 4 ppb by mass or more and 15 ppb by mass or less.

When the content of these impurities is in the above-described numerical range, high-purity hydrocarbon can be produced without an excessive increase in production cost.

As a method of removing the impurities from the crude hydrocarbon for purification, a membrane separation method, an adsorption method, a filtration method, or a distillation method, or the like can be applied. In the method for producing hydrocarbon according to the present embodiment, the high-purity hydrocarbon is obtained through the hydrogen gas removal step of removing hydrogen gas from crude hydrocarbon to obtain low-hydrogen hydrocarbon and the adsorption step of bringing the low-hydrogen hydrocarbon into contact with the adsorbent. A purification step other than the hydrogen gas removal step and the adsorption step may be further combined, and a purification step using any purification method can be selected depending on the kinds and amounts of impurities to be removed, the production capacity of hydrocarbon, and the like.

[Hydrogen Gas Removal Step]

The hydrogen gas removal step is a step of removing hydrogen gas from the crude hydrocarbon to obtain low-hydrogen hydrocarbon in which a content of hydrogen gas is 100 ppm by volume or less. The method of removing hydrogen gas from the crude hydrocarbon in the hydrogen gas removal step is not particularly limited. For example, a membrane separation method of causing the crude hydrocarbon to pass through a separation membrane or a distillation method of distilling the crude hydrocarbon can be used.

The membrane separation method will be described below in detail. By causing the crude hydrocarbon to pass through the separation membrane, hydrogen gas can be separated from the crude hydrocarbon. In addition, there is a case where nitrogen gas and oxygen gas can also be separated from each other. The material, shape, pore diameter, and the like of the separation membrane to be used are not particularly limited as long as there can be a difference in permeability rate between hydrocarbon and impurities.

Examples of a material of the separation membrane include polyethylene, polypropylene, polyvinylidene fluoride, polysulfone, polyethersulfone, cellulose acetate, polyimide, alumina, mullite, and titania. Among these materials, polyimide is preferable from the viewpoint of easy availability.

Examples of the shape of the separation membrane include a hollow fiber shape, a cylindrical shape, and a corrugated shape. Among these shapes, a hollow fiber shape is preferable from the viewpoint of easy availability.

In general, the permeability rate at which hydrocarbon permeates through the separation membrane is low, and the permeability rate at which the gaseous impurities such as hydrogen gas permeate through the separation membrane is high. Therefore, the low-hydrogen hydrocarbon from which the impurities are removed can be collected from a non-permeated gas outlet of the separation membrane. That is, gas that does not permeate through the separation membrane in the crude hydrocarbon is the low-hydrogen hydrocarbon.

The purification method using the separation membrane can be applied to removal of any impurity. From the viewpoint that the rate at which gas permeates through the separation membrane is fast, it is effective to remove hydrogen gas, nitrogen gas, and oxygen gas, and it is more effective to remove hydrogen gas.

Conditions of the membrane separation method such as a temperature or a pressure can be freely set as long as hydrocarbon and impurities can be separated and a temperature condition and a pressure condition that can be used for the separation membrane are satisfied.

The temperature condition is, for example, preferably -20°C or higher and 200°C or lower, more preferably 0°C or higher and 150°C or lower, still more preferably 5°C or higher and 80°C or lower, and particularly preferably 10°C or higher and 40°C or lower.

In addition, the pressure condition is preferably 0.1 MPa or more and 5 MPa or less, more preferably 0.15 MPa or more and 4 MPa or less, and still more preferably 0.2 MPa or more and 3 MPa or less.

[Adsorption Step]

The adsorption step is a step of bringing the low-hydrogen hydrocarbon into contact with an adsorbent to obtain high-purity hydrocarbon in which a content of hydrogen gas is 80 ppm by volume or less, a total content of nitrogen gas and oxygen gas is 5 ppm by volume or less, and a total content of boron, aluminum, phosphorus, sulfur, titanium, vanadium, chromium, and molybdenum is 250 ppb by mass or less. The adsorbent used in the adsorption step includes a crystal having pores with a pore diameter of more than 0.3 nm and 3.5 nm or less. A crystal form of the crystal in the adsorbent is not a mordenite type.

In the adsorption step, at least one among nitrogen gas and oxygen gas may be adsorbed on the adsorbent from the low-hydrogen hydrocarbon, or at least one among boron, aluminum, phosphorus, sulfur, titanium, vanadium, chromium, and molybdenum may be adsorbed on the adsorbent from the low-hydrogen hydrocarbon. Alternatively, all of nitrogen gas, oxygen gas, boron, aluminum, phosphorus, sulfur, titanium, vanadium, chromium, and molybdenum may be adsorbed on the adsorbent from the low-hydrogen hydrocarbon.

The kind of the adsorbent is not particularly limited as long as the above-described impurities can be adsorbed. When the pore diameter (average pore diameter) of the pores in the adsorbent is excessively small, desorption of nitrogen gas and oxygen gas is likely to occur. On the other hand, when the pore diameter (average pore diameter) of the pores in the adsorbent is excessively large, there is a concern that the productivity of the high-purity hydrocarbon may decrease because an excessive amount of hydrocarbon is adsorbed, and there is a concern that the adsorption power of nitrogen gas and oxygen gas may be insufficient.

Therefore, the pore diameter (average pore diameter) of the pores in the adsorbent needs to be more than 0.3 nm and 3.5 nm or less and is preferably 0.4 nm or more and 2.5 nm or less and more preferably 0.5 nm or more and 1 nm or less. By using the adsorbent, nitrogen gas and oxygen gas in the low-hydrogen hydrocarbon are sufficiently adsorbed. The pore diameter (average pore diameter) of the pores can be measured using a gas adsorption method (for example, a BET adsorption method).

The material for forming the adsorbent is not particularly limited, and from the viewpoint of economic efficiency or easy availability, activated carbon, zeolite, silica gel, or alumina is preferable, and activated carbon or zeolite is more preferable. As the activated carbon, coconut shell activated carbon is preferable.

As the zeolite, a zeolite that includes a crystal having a crystal form of an A-type, X-type, Y-type, L-type, or ferrierite-type can be used, and the crystal form is preferably the A-type or the X-type. On the other hand, the zeolite having a crystal form of the mordenite type is not preferable because the adsorption capacity of nitrogen gas and oxygen gas is not sufficient even when the pore diameter (average pore diameter) of the pores is more than 0.3 nm and 3.5 nm or less.

Adsorption conditions such as a temperature or a pressure are not particularly limited as long as the impurities can be adsorbed. When the temperature is low and the pressure is high, nitrogen gas and oxygen gas are likely to be adsorbed on the adsorbent, and hydrocarbon is also likely to be adsorbed on the adsorbent. When the temperature is excessively high, there is a concern that nitrogen gas and oxygen gas may be desorbed from the adsorbent.

Therefore, the temperature condition is, for example, preferably -20°C or higher and 200°C or lower, more preferably 0°C or higher and 150°C or lower, still more preferably 5°C or higher and 80°C or lower, and particularly preferably 10°C or higher and 40°C or lower.

In addition, the pressure condition is preferably 0.1 MPa or more and 5 MPa or less, more preferably 0.15 MPa or more and 4 MPa or less, and still more preferably 0.2 MPa or more and 3 MPa or less. When the pressure is in the above-described range, nitrogen gas, oxygen gas, boron, aluminum, phosphorus, sulfur, titanium, vanadium, chromium, and molybdenum are likely to be adsorbed on the adsorbent from the low-hydrogen hydrocarbon, and the contents of these impurities are likely to be sufficiently reduced.

[Filtration Step]

The method for producing hydrocarbon according to the present embodiment may further include a filtration step of causing hydrocarbon to pass through a filter medium to filter and remove solid impurities. That is, the method may further include a filtration step of causing the crude hydrocarbon, the low-hydrogen hydrocarbon, or the high-purity hydrocarbon to pass through a filter medium to remove at least one among boron, aluminum, phosphorus, sulfur, titanium, vanadium, chromium, and molybdenum from the crude hydrocarbon, the low-hydrogen hydrocarbon, and the high-purity hydrocarbon.

By performing the filtration step, the contents of boron, aluminum, phosphorus, sulfur, titanium, vanadium, chromium, and molybdenum in the hydrocarbon can be further reduced.

The filtration step can be performed on the crude hydrocarbon, the low-hydrogen hydrocarbon, or the high-purity hydrocarbon as described above. The filtration step may be performed on any one of the three hydrocarbons, may be performed on two of the three hydrocarbons, or may be performed on all of the three hydrocarbons. For example, the filtration step may be performed only after the adsorption step, or the filtration step may be performed three times in total before and after the hydrogen gas removal step and after the adsorption step.

The kind of the filter medium is not particularly limited as long as at least one among boron, aluminum, phosphorus, sulfur, titanium, vanadium, chromium, and molybdenum can be removed. For example, a filter, powder, a pellet, or the like capable of increasing the contact area of the impurities and the filter medium can be used as the filter medium.

Among these, a filter medium having a filter shape with low pressure loss is preferably used, and at least one filter among a membrane filter (for example, a membrane filter formed of a resin), a sintered metal filter, and a filter including a plurality of metal halide pellets is more preferably used.

The kind of the resin for forming the membrane filter is not particularly limited, and examples thereof include polyethylene, polypropylene, nylon, polytetrafluoroethylene (PTFE), polychlorotrifluoroethylene (PCTFE), and a tetrafluoroethylene-perfluoroalkyl vinyl ether copolymer (PFA).

The kind of the metal for forming the sintered metal filter is not particularly limited, and examples thereof include stainless steel and a nickel-based alloy. Examples of the stainless steel include austenitic stainless steel, martensitic stainless steel, and ferritic stainless steel. Among these, easily available austenitic stainless steel is preferable, and SUS304, SUS304L, SUS316, SUS316L, or the like is more preferable. Examples of the nickel-based alloy include nickel, Monel (registered trademark), and Hastelloy (registered trademark). Among these, easily available nickel is preferable.

The configuration of the filter including a plurality of metal halide pellets is not particularly limited as long as solid impurities can be filtered and removed.

The kind of the metal halide for forming the pellets of the filter including a plurality of metal halide pellets is not particularly limited, and examples thereof include a metal fluoride, a metal chloride, a metal bromide, and a metal iodide. Among these, the metal fluoride is preferable. Examples of the metal species included in the metal halide include lithium (Li), sodium (Na), potassium (K), cesium (Cs), magnesium (Mg), calcium (Ca), iron (Fe), cobalt (Co), nickel (Ni), and aluminum. Among these, sodium, potassium, magnesium, or aluminum that is easily available is preferable, and sodium is more preferable.

Filtration conditions such as a temperature or a pressure are not particularly limited as long as the impurities can be filtered. The temperature condition is, for example, preferably -20°C or higher and 200°C or lower, more preferably 0°C or higher and 150°C or lower, still more preferably 5°C or higher and 80°C or lower, and particularly preferably 10°C or higher and 40°C or lower.

In addition, the pressure condition is preferably 0.1 MPa or more and 5 MPa or less, more preferably 0.15 MPa or more and 4 MPa or less, and still more preferably 0.2 MPa or more and 3 MPa or less.

When the temperature condition and the pressure condition are in the above-described range, the solid impurities are likely to be removed from hydrocarbon without requiring much effort.

Next, the method for producing silicon carbide according to the present embodiment will be described. For example, a method for producing a silicon carbide epitaxial wafer by using, as a raw material, high-purity hydrocarbon produced with the method for producing hydrocarbon according to the present embodiment will be described. The method for producing a silicon carbide epitaxial wafer described below includes a polishing step, a cleaning (gas etching) step, a film forming (epitaxial growth) step, and a temperature decrease step. These steps will be described.

[Polishing Step]

First, a hexagonal silicon carbide single-crystal substrate is prepared and polished using a polishing method such as chemical machine polishing until the thickness of a lattice disorder layer on the surface is 3 nm or less. "Lattice disorder layer" refers to a layer where a striped structure corresponding to an atomic layer (lattice) of the silicon carbide single crystal and a part of the stripes is not clear in a lattice image (image where a lattice can be verified) obtained using a transmission electron microscope (TEM).

The kind of abrasive grains in a polishing solution used for polishing is not particularly limited, and it is preferable to use particles that are dispersed without being dissolved in the pH range of the polishing solution. The pH of the polishing solution is preferably less than **2**. In this case, particles of diamond, silicon carbide, aluminum oxide, titanium oxide, silicon oxide, or the like can be used as the abrasive grains.

The average particle diameter of the abrasive grains is not particularly limited and is preferably 1 nm or more to 400 nm or less, more preferably 10 nm or more to 200 nm or less, and still more preferably 10 nm or more to 150 nm or less.

To obtain an excellent finally finished surface, from the viewpoint that abrasive grains having a small average particle diameter are commercially available at a low cost, silica abrasive grains are preferable, and colloidal silica abrasive grains are more preferable. The average particle diameter of the colloidal silica abrasive grains can be appropriately selected depending on processing characteristics such as a processing speed or surface roughness.

When a higher polishing rate is required, abrasive grains having a large average particle diameter may be used. When a low surface roughness, that is, a highly smooth surface is required, abrasive grains having a small average particle diameter may be used. Abrasive grains having an average particle diameter of more than 400 nm are expensive but have a slow polishing rate, which is not economical. In abrasive grains having an extremely small average particle diameter of less than 1 nm, the polishing rate significantly decreases.

The content of the abrasive grains in the polishing solution is not particularly limited and is preferably 1% by mass or more and 30% by mass or less and more preferably 1.5% by mass or more and 15% by mass or less. When the content of the abrasive grains in the polishing solution is in the above-described numerical range, it is difficult to dry the abrasive grains, which is advantageous in that, for example, scratches are not likely to be formed, the configuration is economical, and the processing speed is fast.

The polishing solution may be an aqueous slurry, and the pH at 20°C is preferably less than 2.0, more preferably less than 1.5, and still more preferably less than 1.2. When the pH of the polishing solution is in the above-described numerical range, a sufficient polishing rate can be obtained. In addition, when the pH of the polishing solution is in the above-described numerical range, chemical reactivity to silicon carbide significantly increases even in a normal indoor environment, and thus ultra precision polishing can be performed.

It is considered that, without directly removed and polished by a mechanical action of the abrasive grains such as oxide particles in the polishing solution, silicon carbide is polished by a mechanism in which the polishing solution chemically reacts with a silicon carbide single crystal surface to form silicon oxide and the formed silicon oxide is mechanically removed by the abrasive grains. Accordingly, to obtain a smooth surface having no scratches or no affected layer, it is important to adjust the liquidity of the polishing solution such that silicon carbide is easily reactive, that is, to adjust the pH to less than 2 and to select particles (for example, oxide particles) having an appropriate hardness as the abrasive grains.

The pH of the polishing solution may be adjusted using an acid such as hydrochloric acid, nitric acid, phosphoric acid, or sulfuric acid. The acid to be used may be used alone or is preferably used in combination of two or more kinds. The reason why the use of plural kinds of acids is effective is not clear but is experimentally verified, and there is a possibility that the plural kinds of acids may react with each other to improve the effect.

For example, the content of the acid in the polishing solution may be 0.5% by mass or more and 5% by mass or less in the case of hydrochloric acid, may be 0.5% by mass or more and 5% by mass or less in the case of nitric acid, may be 0.5% by mass or more and 5% by mass or less in the case of phosphoric acid, and 0.5% by mass or more and 5% by mass or less in the case of sulfuric acid. The kinds and the contents of the acids may be determined in the above-described ranges to adjust the pH to less than 2.

The reason why the use of an inorganic acid is effective is that the liquidity of the polishing solution is easily adjusted to be strongly acidic because the inorganic acid is stronger than an organic acid. It is not easy to adjust the liquidity of the polishing solution to be strongly acidic using the organic acid.

The polishing of silicon carbide is performed by removing an oxide film with the abrasive grains, the oxide film being formed on the surface of the silicon carbide by the strongly acidic polishing solution. To accelerate the surface oxidation, an oxidant may be added to the polishing solution. The kind of the oxidant is not particularly limited, and examples thereof include hydrogen peroxide, perchloric acid, potassium dichromate, and ammonium persulfate. For example, when hydrogen peroxide water is used, the content thereof in the polishing solution is 0.5% by mass or more and 5% by mass or less and preferably 1.5% by mass or more and 4% by mass or less from the viewpoint of improving the polishing rate.

To suppress gelation of the abrasive grains, an antigelling agent may be added to the polishing solution. The kind of the antigelling agent is not particularly limited. For example, a phosphoric acid ester-based chelating agent such as 1-hydroxyethylidene-1,1-diphosphonic acid or aminotriethylene phosphonic acid is suitably used. The content of the antigelling agent in the polishing solution is not particularly limited and is preferably 0.01% by mass or more and 6% by mass or less and more preferably 0.05% by mass or more and 2% by mass or less.

[Cleaning Step]

In the cleaning step, the polished hexagonal silicon carbide single-crystal substrate is held at a high temperature in a hydrogen gas atmosphere to clean (gas-etch) the surface. An example of the cleaning step will be described below.

The hexagonal silicon carbide single-crystal substrate polished in the polishing step is cleaned and subsequently provided in an epitaxial growth device (for example, a mass-production type multi-wafer planetary CVD device). Hydrogen gas is introduced into the epitaxial growth device, and the pressure in the epitaxial growth device is adjusted to be 100 mbar or more and 250 mbar or less. The flow rate of hydrogen gas is 40 slm or more and 120 slm or less.

By increasing the temperature in the epitaxial growth device such that the temperature of the hexagonal silicon carbide single-crystal substrate is 1400°C or higher and 1600°C or lower and preferably 1480°C or higher and 1600°C or lower, the surface of the hexagonal silicon carbide single-crystal substrate is cleaned by the hydrogen gas. The treatment time is 5 minutes or longer and 30 minutes or shorter. When cleaning is performed under this condition, the etching amount is 0.05 $\mu$m or more and 0.4 $\mu$m or less.

[Film Forming Step]

In the film forming step, silane ($SiH_4$) gas and propane gas of amounts required for epitaxial growth of silicon carbide are supplied to the surface of the hexagonal silicon carbide single-crystal substrate cleaned in the cleaning step to epitaxially grow silicon carbide.

Conditions of the film forming step may be determined depending on a desired film thickness and a desired film growth rate. For example, the flow rate of silane gas may be 15 sccm or more and 150 sccm or less, the flow rate of propane gas may be 3.5 sccm or more and 60 sccm or less, the pressure may be 80 mbar or more and 250 mbar or less, and the substrate temperature may be 1400°C or higher and 1600°C or lower.

As a method of suppressing step bunching during growth of an epitaxial film of silicon carbide, a method of controlling a quantitative ratio between raw material gases supplied to increase migration of silicon atoms on the growth surface is known. In the present embodiment, it is preferable that a ratio between the supply amounts of silane gas and propane gas is controlled such that a molar ratio C/Si of carbon atoms in the propane to silicon atoms in the silane is 0.7 or more and 1.2 or less.

In addition, the growth rate of the epitaxial film of silicon carbide may be 3 $\mu$m/h or faster and 20 $\mu$m/h or slower. Typically, the film thickness of the epitaxial film of silicon carbide to be grown is 5 $\mu$m or more and 20 $\mu$m or less.

[Temperature Decrease Step]

In the film forming step, silane gas and propane gas are supplied into the epitaxial growth device. After the formation of epitaxial film of silicon carbide ends, the supply of silane gas and propane gas is stopped simultaneously, and the supply of nitrogen gas introduced as doping gas is also stopped.

The temperature of the hexagonal silicon carbide single-crystal substrate is held until the exhaust of silane gas and propane gas from the epitaxial growth device is completed, and the temperature of the hexagonal silicon carbide single-crystal substrate is decreased after the exhaust is completed (temperature decrease step).

Note that, even in the temperature decrease step, gas etching may occur on the surface of the epitaxial film of silicon carbide such that morphology of the surface deteriorates. To suppress the deterioration of the morphology of the surface, the timing at which the supply of silane gas and propane gas is stopped and the timing of the temperature decrease are important.

After the supply of silane gas and propane gas is simultaneously stopped, the growth temperature is held until the supplied silane gas and the supplied propane gas are removed from the surface of the hexagonal silicon carbide single-crystal substrate. Next, when the temperature decreases to normal temperature at an average rate of about 50 °C/min, the deterioration of the morphology of the surface of the epitaxial film of silicon carbide can be suppressed.

Examples

Hereinafter, the present invention will be described more specifically by describing Examples and Comparative Examples.

[Example 1-1]

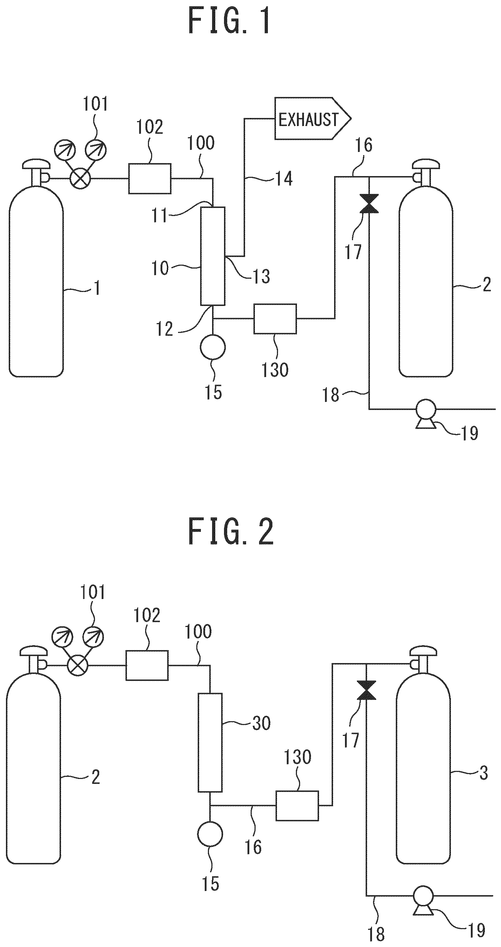

Propane (1) that was crude hydrocarbon was purified using a membrane separation device illustrated in FIG. 1. A cylinder 1 (volume: 47 L, formed of manganese steel) filled with the propane (1) is connected to a gas inlet 11 of a separation membrane module 10 (CO-B01, manufactured by UBE Corporation) through a pipe 100 formed of SUS316. In the pipe 100, a pressure control device 101 and a mass flow controller 102 are provided in series in this order from the upstream side to the downstream side.

The separation membrane module 10 includes a separation membrane (not illustrated), a non-permeated gas outlet 12 from which gas that does not permeate through the separation membrane is exhausted, and a permeated gas outlet 13 from which gas that permeates through the separation membrane is exhausted. The permeated gas outlet 13 is connected to a combustion abatement device (not illustrated) through an exhaust pipe 14. The non-permeated gas outlet 12 is connected to a cylinder 2 (volume: 47 L, formed of manganese steel) through a collection pipe 16 formed of SUS316. In the collection pipe 16, a flow meter 130 is provided.

A branch pipe is branched two ways and extends from a portion between the separation membrane module 10 and the flow meter 130 in the collection pipe 16, and the tip of the branch pipe is connected to a pressure gauge 15. In addition, an evacuation pipe 18 formed of SUS316 is branched two ways and extends from a portion between the flow meter 130 and the cylinder 2 in the collection pipe 16. In the evacuation pipe 18, a vacuum pump connection valve 17 and a vacuum pump 19 are provided in series in this order from the upstream side to the downstream side.

The flow rate of the gas flowing out from the non-permeated gas outlet 12 can be measured by the flow meter 130. An exhaust port of the vacuum pump 19 is connected to a local exhaust ventilation facility (not illustrated). The cylinder 2 can be cooled to any temperature by a cooling tank (not illustrated).

The propane (1) filled in the cylinder 1 was caused to flow through the separation membrane module 10 under conditions of a temperature of 30°C, a pressure of 0.4 MPaG, and a flow rate of 1000 mL/min. 7 kg of the gas exhausted from the non-permeated gas outlet 12 was filled in the cylinder 2 that was cooled to -78°C by the cooling tank (not illustrated) and was depressurized to an inner pressure of 1 kPa or lower to produce propane (2). This propane (2) is low-hydrogen hydrocarbon obtained by purifying the propane (1) using the separation membrane.

The flow rate of the gas flowing out from the non-permeated gas outlet 12 was 710 mL/min. The gas exhausted from the permeated gas outlet 13 was treated by the combustion abatement device (not illustrated) and exhausted through the exhaust pipe 14.

Next, the propane (2) was purified using an adsorption device illustrated in FIG. 2. The configuration of the adsorption device of FIG. 2 is substantially the same configuration as that of the membrane separation device of FIG. 1, except that the cylinder 1 was changed to the cylinder 2, that the cylinder 2 was changed to a cylinder 3, and that the separation membrane module 10 was changed to an adsorption tower 30.

The propane (2) filled in the cylinder 2 was caused to flow through the adsorption tower 30 filled with an adsorbent under conditions of a temperature of 30°C, a pressure of 0.4 MPaG, and a flow rate of 1000 mL/min. 2 kg of the gas passed through the adsorption tower 30 was filled in the cylinder 3 (volume: 47L, formed of manganese steel) that was cooled to -78°C by the cooling tank (not illustrated) and was depressurized to an inner pressure of 1 kPa or lower to produce propane (3). This propane (3) is high-purity hydrocarbon obtained by purifying the propane (2) using the adsorbent.

The flow rate of outlet gas of the adsorption tower 30 was 310 mL/min. Regarding the dimension of the adsorption tower 30, the inner diameter was 100 mm and the length was 2000 mm. The adsorbent is MOLECULAR SIEVE 13X (X-type zeolite, average pore diameter: 1 nm) manufactured by Union Showa K.K., and the amount of the adsorbent filled in the adsorption tower 30 is 7kg.

The average pore diameter of the adsorbent was measured as follows.

Measuring device: Belsorp max manufactured by Nikkiso Co., Ltd.
Adsorbate: nitrogen gas
Measurement temperature: 77 K

Sample pretreatment: heating and drying at 300°C for 6 h under a vacuum condition
Amount of sample: 0.10 g

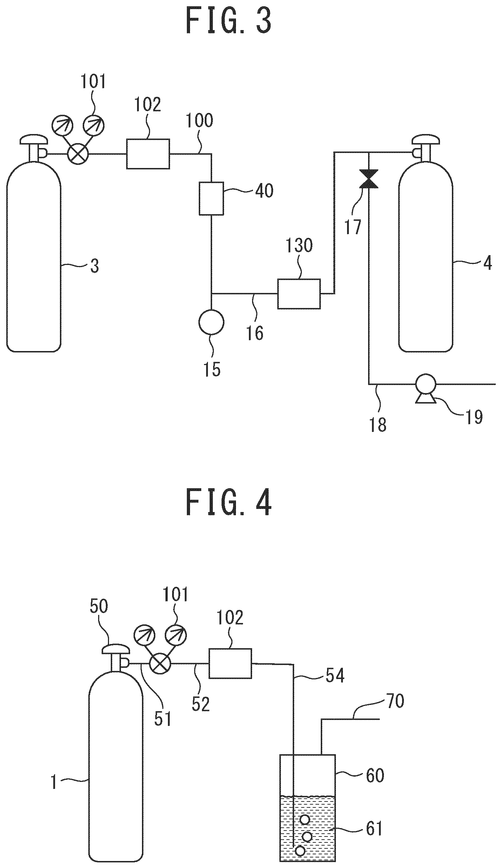

Next, the propane (3) was purified using a filtration device illustrated in FIG. 3. The configuration of the filtration device of FIG. 3 is substantially the same configuration as that of the membrane separation device of FIG. 1, except that the cylinder 1 was changed to the cylinder 3, and that the cylinder 2 was changed to a cylinder 4, and that the separation membrane module 10 was changed to a gas filter 40.

The propane (3) filled in the cylinder 3 was caused to flow through the gas filter 40 under conditions of a temperature of 30°C, a pressure of 0.4 MPaG, and a flow rate of 1000 mL/min. 1 kg of the gas passed through the gas filter 40 was filled in the cylinder 4 (volume: 47L, formed of manganese steel) that was cooled to -78°C by the cooling tank (not illustrated) and was depressurized to an inner pressure of 1 kPa or lower to produce propane (4). This propane (4) is propane gas purified by filtering the propane (3) through a filter medium.

The flow rate of outlet gas of the gas filter 40 was 1000 mL/min. The gas filter 40 is a membrane filter formed of PTFE, which is Wafergard (registered trademark) manufactured by Entegris Japan Co., Ltd.

Next, the analysis of the propanes (1) to (4) was performed to measure the content of hydrogen gas. Table 1 shows the results. Measurement conditions are as follows.

Analyzer: gas chromatography (Tracera manufactured by Shimadzu Corporation)
Detector: TCD
Temperature of Detector: 150°C
Current Value of Detector: 180 mA
Injection amount of sample: 1.0 mL
Column: Micropacked ST
Temperature of column: 200°C
Carrier gas: argon
Flow rate of carrier gas: 20 mL/min

Next, the analysis of the propanes (1) to (4) was performed to measure the content of nitrogen gas and the content of oxygen gas. Table 1 shows the results. Measurement conditions are as follows.

Analyzer: gas chromatography (Tracera manufactured by Shimadzu Corporation)
Detector: BID
Temperature of Detector: 250°C
Injection amount of sample: 1.0 mL
Column: Micropacked ST
Temperature of column: 200°C
Carrier gas: helium
Flow rate of carrier gas: 9.0 mL/min

The analysis of the propanes (1) to (4) was performed to measure the content of boron, aluminum, phosphorus, sulfur, titanium, vanadium, chromium, and molybdenum. Table 1 shows the results. The measurement method will be described using the propane (1) as an example, but the same can be substantially applied to the propanes (2) to (4).

The cylinder 1 was removed from the membrane separation device of FIG. 1 and was mounted on the preparation device of FIG. 4. That is, a valve 50 of the cylinder 1 is connected to the pressure control device 101 through a pipe 51, and the pressure control device 101 is connected to the mass flow controller 102 through a connection pipe 52. The mass flow controller 102 is connected to a nitric acid container 60 through a connection pipe 54. The nitric acid container 60 accommodates 100 g of a nitric acid aqueous solution 61 having a concentration of 1% by mass, and the tip of the connection pipe 54 is disposed in the nitric acid aqueous solution 61. In addition, an exhaust port 70 is provided in the nitric acid container 60.

While vaporizing liquid propane in the cylinder (1) at 20°C, propane gas was taken out from the gas phase portion in the cylinder (1), was caused to flow through the nitric acid aqueous solution 61 in the nitric acid container 60 at a flow rate of 100 mL/min, and was bubbled. By bringing the propane gas into contact with the nitric acid aqueous solution 61, boron, aluminum, phosphorus, sulfur, titanium, vanadium, chromium, and molybdenum in the propane gas were absorbed on the nitric acid aqueous solution 61.

The mass (M1) of the nitric acid aqueous solution 61 after the flow of the propane gas was 96 g. A difference in the mass (M2) of the cylinder 1 before and after the flow of the propane gas was 10 g. The nitric acid aqueous solution 61 was diluted with ultrapure water such that the mass was 100 g (M3), the diluted nitric acid aqueous solution was collected, and the analysis of the metals was performed using an inductively coupled plasma mass spectrometer to measure each of signal intensities (y) of boron, aluminum, phosphorus, sulfur, titanium, vanadium, chromium, and molybdenum in the diluted nitric acid aqueous solution.

The contents (M) of boron, aluminum, phosphorus, sulfur, titanium, vanadium, chromium, and molybdenum were calculated from the signal intensities (y) using a calibration curve, respectively to obtain the total content of boron, aluminum, phosphorus, sulfur, titanium, vanadium, chromium, and molybdenum.

The contents (M) of boron, aluminum, phosphorus, sulfur, titanium, vanadium, chromium, and molybdenum in the propane (1) can be calculated from the following formula.

$$M = [\{y - b)/a\} \times (M3/M1)]/M2$$

In the above-described formula, a represents the slope of the calibration curve, and b represents the intercept of the calibration curve. The used calibration curve was formed as follows. That is, nitric acid standard solutions where the contents of the above-described respective impurities were 0 ppb by mass (not containing the impurities), 10 ppb by mass, 100 ppb by mass, 300 ppb by mass, and 700 ppb by mass, respectively, were produced and were analyzed using an inductively coupled plasma mass spectrometer. The content of the impurities in the nitric acid standard solution was represented on the horizontal axis, the calibration curve obtained by plotting the signal intensities was represented on the vertical axis, and the slope (a) and the intercept (b) thereof were obtained.

[Table 1]

| | | Flow Rate (mL/min) | | Contents of Gaseous Impurities (ppm by volume) | | | Contents of Solid Impurities (ppb by mass) | | | | | | | | |
| --- | --- | --- | --- | --- | --- | --- | --- | --- | --- | --- | --- | --- | --- | --- | --- |
| | | Inlet | Outlet | Hydrogen Gas | Nitrogen Gas | Oxygen Gas | B | Al | P | S | Ti | V | Cr | Mo | Total |
| Ex. 1-1 | Propane (1) | - | - | 1201 | 14 | 5 | 58 | 18 | 97 | 82 | 92 | 31 | 101 | 37 | 516 |
| | Propane (2) | 1000 | 730 | 97 | 8 | 2 | 41 | 12 | 36 | 33 | 33 | 17 | 28 | 15 | 215 |
| | Propane (3) | 1000 | 310 | 74 | 0.5 | 0.5 | 12 | 9 | 10 | 15 | 14 | 5 | 13 | 6 | 84 |
| | Propane (4) | 1000 | 1000 | 74 | 0.5 | 0.5 | 3 | 2 | 3 | 1 | 1 | 1 | 2 | 1 | 14 |
| Ex. 1-2 | Propane (5) | 1000 | 1000 | 74 | 0.5 | 0.5 | 2 | 2 | 3 | 3 | 1 | 1 | 1 | 1 | 14 |
| Ex. 1-3 | Propane (6) | 1000 | 1000 | 74 | 0.5 | 0.5 | 1 | 1 | 2 | 1 | 1 | 1 | 1 | 1 | 9 |
| Ex. 1-4 | Propane (7) | 1000 | 1000 | 63 | 4 | 1 | 14 | 12 | 12 | 10 | 8 | 4 | 6 | 8 | 74 |
| Ex. 1-5 | Propane (8) | 1000 | 250 | 51 | 2 | 1 | 11 | 8 | 10 | 2 | 15 | 10 | 16 | 8 | 80 |
| Ex. 1-6 | Propane (9) | 1000 | 310 | 42 | Less than 0.2 | Less than 0.2 | 8 | 9 | 8 | 7 | 9 | 5 | 3 | 4 | 53 |
| Ex. 1-7 | Propane (10) | 1000 | 280 | 51 | 0.5 | 0.5 | 3 | 2 | 2 | 1 | 1 | 1 | 2 | 1 | 13 |
| Comp. Ex. 1-1 | Propane (11) | 1000 | 1000 | 25 | 21 | 5 | 54 | 13 | 55 | 4 | 30 | 12 | 18 | 9 | 195 |
| Comp. Ex. 1-2 | Propane (12) | 1000 | 80 | 97 | 32 | 6 | 54 | 18 | 75 | 10 | 13 | 8 | 22 | 5 | 205 |
| Comp. Ex. 1-3 | Propane (13) | 1000 | 350 | 441 | 10 | 3 | 13 | 21 | 62 | 43 | 44 | 15 | 108 | 15 | 321 |

EP 4 620 938 A1

[Example 1-2]

Propane (5) was obtained by purifying the propane (1) using the same method as that of Example 1-1, except that a filtration cylinder (inner diameter: 22.9 mm, length: 100 mm) filled with 70 g of a sodium fluoride pellet (a cylindrical pellet having a diameter of 3 mm and a length of 5 mm, manufactured by Morita Chemical Industries Co., Ltd.) was used as the gas filter 40 instead of the membrane filter formed of PTFE.

The contents of hydrogen gas, nitrogen gas, oxygen gas, boron, aluminum, phosphorus, sulfur, titanium, vanadium, chromium, and molybdenum in the propane (5) were measured using the same method as that of Example 1-1. Table 1 shows the results.

[Example 1-3]

Propane (6) was obtained by purifying the propane (1) using the same method as that of Example 1-1, except that a nickel membrane filter (Wafergard III, manufactured by Entegris Japan Co., Ltd.) was used as the gas filter 40 instead of the membrane filter formed of PTFE.

The contents of hydrogen gas, nitrogen gas, oxygen gas, boron, aluminum, phosphorus, sulfur, titanium, vanadium, chromium, and molybdenum in the propane (6) were measured using the same method as that of Example 1-1. Table 1 shows the results.

[Example 1-4]

Propane (7) was obtained by purifying the propane (1) using the same method as that of Example 1-1, except that MOLECULAR SIEVE 4A (A-type zeolite, average pore diameter: 0.4 nm) manufactured by Union Showa K.K. was used as the adsorbent filled in the adsorption tower 30 and that the filtration using the gas filter 40 was not performed.

The contents of hydrogen gas, nitrogen gas, oxygen gas, boron, aluminum, phosphorus, sulfur, titanium, vanadium, chromium, and molybdenum in the propane (7) were measured using the same method as that of Example 1-1. Table 1 shows the results.

[Example 1-5]

Propane (8) was obtained by purifying the propane (1) using the same method as that of Example 1-1, except that coconut shell activated carbon (average pore diameter: 2.5 nm) manufactured by Osaka Gas Chemicals Co., Ltd. was used as the adsorbent filled in the adsorption tower 30 and that the filtration using the gas filter 40 was not performed.

The contents of hydrogen gas, nitrogen gas, oxygen gas, boron, aluminum, phosphorus, sulfur, titanium, vanadium, chromium, and molybdenum in the propane (8) were measured using the same method as that of Example 1-1. Table 1 shows the results.

[Example 1-6]

Propane (9) was obtained by purifying the propane (3) obtained in Example 1-1 again using the adsorption device of FIG. 2. That is, the propane (3) was purified by causing the propane (3) to flow through the adsorption tower 30 used in Example 1-1.

The contents of hydrogen gas, nitrogen gas, oxygen gas, boron, aluminum, phosphorus, sulfur, titanium, vanadium, chromium, and molybdenum in the propane (9) were measured using the same method as that of Example 1-1. Table 1 shows the results.

[Example 1-7]

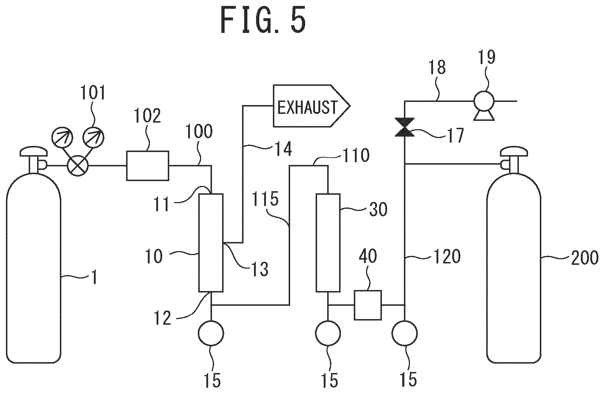

Propane (10) filled in a cylinder 200 was obtained by purifying the propane (1) using a device for producing hydrocarbon illustrated in FIG. 5. In the production device of FIG. 5, the devices of FIGS. 1 to 3 are connected in series, and the membrane separation, the adsorption, and the filtration can be continuously performed using completely the same method as that of Example 1-1 (that is, the operation of filling the propane purified in each of the steps in the cylinder and providing the propane to the next step is not necessary). The device of FIG. 1 and the device of FIG. 2 are connected through a pipe 115, and the device of FIG. 2 and the device of FIG. 3 are connected through a pipe 120.

The contents of hydrogen gas, nitrogen gas, oxygen gas, boron, aluminum, phosphorus, sulfur, titanium, vanadium, chromium, and molybdenum in the propane (10) were measured using the same method as that of Example 1-1. Table 1 shows the results.

[Comparative Example 1-1]

Propane (11) was obtained by purifying the propane (1) using the same method as that of Example 1-1, except that MOLECULAR SIEVE 3A (A-type zeolite, average pore diameter: 0.3 nm) manufactured by Union Showa K.K. was used as the adsorbent filled in the adsorption tower 30 and that the filtration using the gas filter 40 was not performed.
The contents of hydrogen gas, nitrogen gas, oxygen gas, boron, aluminum, phosphorus, sulfur, titanium, vanadium, chromium, and molybdenum in the propane (11) were measured using the same method as that of Example 1-1. Table 1 shows the results.

[Comparative Example 1-2]

Propane (12) was obtained by purifying the propane (1) using the same method as that of Example 1-1, except that coal activated carbon (average pore diameter: 3.7 nm) manufactured by Osaka Gas Chemicals Co., Ltd. was used as the adsorbent filled in the adsorption tower 30 and that the filtration using the gas filter 40 was not performed.
The contents of hydrogen gas, nitrogen gas, oxygen gas, boron, aluminum, phosphorus, sulfur, titanium, vanadium, chromium, and molybdenum in the propane (12) were measured using the same method as that of Example 1-1. Table 1 shows the results.

[Comparative Example 1-3]

Propane (13) was obtained by purifying the propane (1) using the same method as that of Example 1-1, except that the membrane separation using the separation membrane module 10 was not performed and that the filtration using the gas filter 40 was not performed.
The contents of hydrogen gas, nitrogen gas, oxygen gas, boron, aluminum, phosphorus, sulfur, titanium, vanadium, chromium, and molybdenum in the propane (13) were measured using the same method as that of Example 1-1. Table 1 shows the results.

[Examples 2 to 6]

The above-described purified hydrocarbons were produced by performing the purification using the same method as that of Example 1-1, except that ethylene, methane, ethane, n-butane, or isobutane was used instead of propane. The contents of hydrogen gas, nitrogen gas, oxygen gas, boron, aluminum, phosphorus, sulfur, titanium, vanadium, chromium, and molybdenum in the each of the above-described purified hydrocarbons were measured using the same method as that of Example 1-1. Tables 2 to 6 show the results.
Ethylene (1) shown in Table 2 corresponds to the propane (1) according to Example 1-1, which is crude hydrocarbon. In addition, ethylene (2) shown in Table 2 corresponds to the propane (2) according to Example 1-1, which is low-hydrogen hydrocarbon obtained by purifying the ethylene (1) through the membrane separation using the separation membrane module 10. Ethylene (3) shown in Table 2 corresponds to the propane (3) according to Example 1-1, which is high-purity hydrocarbon obtained by purifying the ethylene (2) through the adsorption using the adsorption tower 30. Ethylene (4) shown in Table 2 corresponds to the propane (4) according to Example 1-1, which is ethylene obtained by purifying the ethylene (3) through the filtration using the gas filter 40. The same can be applied to methane, ethane, n-butane, and isobutane.

[Table 2]

| | | Flow Rate (mL/min) | | Contents of Gaseous Impurities (ppm by volume) | | | Contents of Solid Impurities (ppb by mass) | | | | | | | | |
| --- | --- | --- | --- | --- | --- | --- | --- | --- | --- | --- | --- | --- | --- | --- | --- |
| | | Inlet | Outlet | Hydrogen Gas | Nitrogen Gas | Oxygen Gas | B | Al | P | S | Ti | V | Cr | Mo | Total |
| Ex. 2 | Ethylene (1) | - | - | 1018 | 10 | 3 | 47 | 23 | 81 | 57 | 101 | 25 | 101 | 53 | 488 |
| | Ethylene (2) | 1000 | 630 | 76 | 7 | 2 | 39 | 12 | 38 | 29 | 42 | 17 | 24 | 12 | 213 |
| | Ethylene (3) | 1000 | 220 | 52 | 0.5 | 0.5 | 13 | 16 | 10 | 6 | 12 | 6 | 11 | 6 | 80 |
| | Ethylene (4) | 1000 | 1000 | 52 | 0.5 | 0.5 | 3 | 2 | 2 | 1 | 1 | 1 | 2 | 1 | 13 |

[Table 3]

| | | Flow Rate (mL/min) | | Contents of Gaseous Impurities (ppm by volume) | | | Contents of Solid Impurities (ppb by mass) | | | | | | | | |
| --- | --- | --- | --- | --- | --- | --- | --- | --- | --- | --- | --- | --- | --- | --- | --- |
| | | Inlet | Outlet | Hydrogen Gas | Nitrogen Gas | Oxygen Gas | B | Al | P | S | Ti | V | Cr | Mo | Total |
| Ex. 3 | Methane (1) | - | - | 1057 | 13 | 3 | 36 | 18 | 62 | 86 | 85 | 18 | 81 | 40 | 426 |
| | Methane (2) | 1000 | 430 | 64 | 8 | 2 | 32 | 12 | 53 | 31 | 36 | 12 | 14 | 13 | 203 |
| | Methane (3) | 1000 | 340 | 47 | 0.5 | 0.5 | 12 | 16 | 10 | 9 | 11 | 5 | 7 | 4 | 74 |
| | Methane (4) | 1000 | 1000 | 47 | 0.5 | 0.4 | 3 | 4 | 2 | 1 | 1 | 1 | 2 | 1 | 15 |
| Comp. Ex. 2 | Methane (5) | 1000 | 720 | 55 | 4 | 3 | 14 | 12 | 32 | 28 | 21 | 9 | 10 | 8 | 134 |

[Table 4]

| | | Flow Rate (mL/min) | | Contents of Gaseous Impurities (ppm by volume) | | | Contents of Solid Impurities (ppb by mass) | | | | | | | | |
| | | Inlet | Outlet | Hydrogen Gas | Nitrogen Gas | Oxygen Gas | B | Al | P | S | Ti | V | Cr | Mo | Total |
|---|---|---|---|---|---|---|---|---|---|---|---|---|---|---|---|
| Ex. 4 | Ethane (1) | - | - | 1201 | 12 | 2 | 44 | 18 | 53 | 91 | 123 | 41 | 100 | 61 | 531 |
| | Ethane (2) | 1000 | 510 | 76 | 8 | 2 | 29 | 12 | 43 | 23 | 57 | 21 | 34 | 25 | 244 |
| | Ethane (3) | 1000 | 420 | 53 | 0.5 | 0.5 | 11 | 16 | 10 | 8 | 16 | 9 | 12 | 5 | 87 |
| | Ethane (4) | 1000 | 1000 | 53 | 0.5 | 0.4 | 2 | 2 | 2 | 3 | 2 | 1 | 2 | 1 | 15 |

[Table 5]

| | | Flow Rate (mL/min) | | Contents of Gaseous Impurities (ppm by volume) | | | Contents of Solid Impurities (ppb by mass) | | | | | | | | | |
| | | Inlet | Outlet | Hydrogen Gas | Nitrogen Gas | Oxygen Gas | B | Al | P | S | Ti | V | Cr | Mo | Total |
|---|---|---|---|---|---|---|---|---|---|---|---|---|---|---|---|
| Ex. 5 | n-Butane (1) | - | - | 1101 | 19 | 4 | 47 | 21 | 56 | 104 | 114 | 32 | 87 | 54 | 515 |
| | n-Butane (2) | 1000 | 890 | 32 | 7 | 2 | 25 | 13 | 39 | 44 | 59 | 15 | 29 | 23 | 247 |
| | n-Butane (3) | 1000 | 560 | 29 | 0.5 | 0.5 | 5 | 16 | 10 | 4 | 23 | 5 | 7 | 3 | 73 |
| | n-Butane (4) | 1000 | 1000 | 29 | 0.5 | 0.4 | 2 | 2 | 3 | 2 | 2 | 1 | 2 | 1 | 15 |

[Table 6]

| | | Flow Rate (mL/min) | | Contents of Gaseous Impurities (ppm by volume) | | | Contents of Solid Impurities (ppb by mass) | | | | | | | | |
|---|---|---|---|---|---|---|---|---|---|---|---|---|---|---|---|
| | | Inlet | Outlet | Hydrogen Gas | Nitrogen Gas | Oxygen Gas | B | Al | P | S | Ti | V | Cr | Mo | Total |
| Ex. 6 | Isobutane (1) | - | - | 1123 | 15 | 4 | 44 | 18 | 53 | 111 | 96 | 29 | 79 | 43 | 473 |
| | Isobutane (2) | 1000 | 930 | 52 | 7 | 2 | 22 | 12 | 43 | 46 | 48 | 15 | 33 | 20 | 239 |
| | Isobutane (3) | 1000 | 950 | 38 | 0.5 | 0.5 | 5 | 16 | 10 | 4 | 17 | 5 | 5 | 4 | 66 |
| | Isobutane (4) | 1000 | 1000 | 38 | 0.5 | 0.4 | 3 | 2 | 2 | 2 | 3 | 1 | 1 | 1 | 15 |

[Comparative Example 2]

Methane (5) was obtained by purifying the methane (2) using the same method as that of Example 1-1, except that the hydrocarbon is methane, that synthetic zeolite HS-642 (cationic species: sodium, mordenite type zeolite, average pore diameter: 0.7 nm) manufactured by FUJIFILM Wako Pure Chemical Corporation was used as the adsorbent filled in the adsorption tower 30, and that the filtration using the gas filter 40 was not performed.

The contents of hydrogen gas, nitrogen gas, oxygen gas, boron, aluminum, phosphorus, sulfur, titanium, vanadium, chromium, and molybdenum in the methane (5) were measured using the same method as that of Example 1-1. Table 3 shows the results.

The following can be seen from the result of Example 1-1. That is, by performing the membrane separation step (hydrogen gas removal step), the adsorption step, and the filtration step in combination, hydrogen gas, nitrogen gas, oxygen gas, boron, aluminum, phosphorus, sulfur, titanium, vanadium, chromium, and molybdenum were able to be removed from the propane.

Hydrogen gas, nitrogen gas, and oxygen gas were able to be removed by the membrane separation step and the adsorption step, the removal of hydrogen gas by the membrane separation step was more effective, and the removal of nitrogen gas and oxygen gas by the adsorption step was more effective.

On the other hand, boron, aluminum, phosphorus, sulfur, titanium, vanadium, chromium, and molybdenum were able to be removed using any of the membrane separation step, the adsorption step, and the filtration step. Note that, when boron, aluminum, phosphorus, sulfur, titanium, vanadium, chromium, and molybdenum were removed using the filtration step, the impurities were able to be reduced to a lower concentration by the membrane separation step and the adsorption step. Further, it can be seen that, since the inlet flow rate and the outlet flow rate of the supplied propane were the same, that is, propane was quantitatively purified, the removal by the filtration step was more effective from the viewpoints of the productivity and the removal efficiency of the impurities.

The following can be seen from the result of Examples 1-2 and 1-3. That is, by performing filtration using a metal fluoride as a filter medium and a metal membrane filter, boron, aluminum, phosphorus, sulfur, titanium, vanadium, chromium, and molybdenum were able to be removed. In particular, when the metal membrane filter was used, the contents of boron, aluminum, phosphorus, sulfur, titanium, vanadium, chromium, and molybdenum in the propane were able to be further reduced.

The following can be seen from the result of Examples 1-4 and 1-5. That is, not only when MOLECULAR SIEVE 13X was used as the adsorbent but also when MOLECULAR SIEVE 4A or the coconut shell activated carbon was used, the impurities were able to be further reduced.

The following can be seen from the result of Example 1-6. That is, by causing the hydrocarbon that was purified once by the adsorbent to further flow through the adsorbent, the contents of hydrogen gas, nitrogen gas, and oxygen gas were able to be further reduced. In addition, the contents of boron, aluminum, phosphorus, sulfur, titanium, vanadium, chromium, and molybdenum were able to be further reduced.

The following can be seen from the result of Example 1-7. That is, even when the membrane separation step, the adsorption step, and the filtration step were continuously performed, the purity of propane was able to be increased without any problem.

The results of Comparative Examples 1-1 and 1-2 showed that, when the average pore diameter of the adsorbent was excessively small or excessively large, the contents of nitrogen gas and oxygen gas in the propane were increased. The result shows that nitrogen gas and oxygen gas adsorbed on the adsorbent were released from the adsorbent by causing propane to flow. In addition, the result showed that, when the average pore diameter of the adsorbent was large, the flowing propane was excessively adsorbed such that the productivity of the propane decreased. The above result showed that an appropriate size is present as the average pore diameter of the adsorbent that can be used for the purification of propane.

It can be seen from the result of Comparative Example 1-3 that, only when propane was treated by an adsorbent having an average pore diameter of more than 0.3 nm and 3.5 nm or less, the total content of nitrogen gas and oxygen gas and the total content of boron, aluminum, phosphorus, sulfur, titanium, vanadium, chromium, and molybdenum were not sufficiently reduced. This showed that, when hydrocarbon excessively contained hydrogen gas, the efficiency of the adsorption step decreased.

The following can be seen from the result of Examples 2 to 6. That is, by performing the membrane separation step, the adsorption step, and the filtration step, hydrogen gas, nitrogen gas, oxygen gas, boron, aluminum, phosphorus, sulfur, titanium, vanadium, chromium, and molybdenum were able to be removed from ethylene, methane, ethane, n-butane, and isobutane without any problem.

The following can be seen from the result of Comparative Example 2. That is, in a case where mordenite type zeolite was used as the adsorbent, even when the content of hydrogen gas in the hydrocarbon was reduced to be 100 ppm by volume or less in the membrane separation step, the adsorption of nitrogen gas and oxygen gas from the hydrocarbon was not sufficiently performed.

[Example 7]

A method of forming a silicon carbide film using the propane (4) will be described. By performing the following step, a silicon carbide film was formed on the surface of the hexagonal silicon carbide single-crystal substrate.

First, by performing the polishing step, the surface of the hexagonal silicon carbide single-crystal substrate was polished. This polishing step was performed in two stages. The first polishing was mechanical polishing and was performed using abrasive grains having a diameter of 5 $\mu$m or less at a processing pressure of 350 g/cm$^2$. The second polishing was chemical machine polishing and was performed using a slurry polishing solution for 30 minutes such that the surface roughness Ra of the hexagonal silicon carbide single-crystal substrate was less than 0.5 nm. The abrasive grains in the polishing solution were silica particles having an average particle diameter of 10 to 150 nm. In addition, the polishing solution contains sulfuric acid, and the pH at 20°C was 1.9.

Next, the polished hexagonal silicon carbide single-crystal substrate was RCA-cleaned and was provided in the epitaxial growth device. The RCA cleaning is a wet cleaning method that is generally used for a silicon wafer, and is a cleaning method of removing organic matter, heavy metal, and particles on the surface of the substrate using a mixed solution of sulfuric acid and hydrogen peroxide water, a mixed solution of ammonia and hydrogen peroxide water, a mixed solution of hydrochloric acid and hydrogen peroxide water, and a hydrogen fluoride aqueous solution.

Next, the cleaning (gas etching) step was performed in the epitaxial growth device. The cleaning step was performed for 10 minutes under conditions of a flow rate of hydrogen gas of 90 slm, a pressure in the reactor of 200 mbar, and a substrate temperature of 1550°C.

After completion of the cleaning step, the silicon carbide epitaxial growth step was performed. In the silicon carbide epitaxial growth step, the flow rate of silane gas was 48 sccm, the flow rate of the propane (4) gas was 17.6 sccm, and the molar ratio C/Si of carbon atoms in the propane to silicon atoms in the silane was 1.1. The pressure in the reactor was set to 200 mbar, the temperature of the substrate was set to 1550°C, and the silicon carbide epitaxial growth step was performed for 2 hours to form a silicon carbide epitaxial film having a thickness of 10 $\mu$m.

As the epitaxial growth device, a Hot Wall SiC CVD reactor (manufactured by Aixtron SE) that was a mass-production type multi-wafer planetary CVD device was used. The silicon carbide epitaxial film was grown on the silicon surface inclined by 4° in the <11-20> orientation with respect to the (0001) plane of the hexagonal silicon carbide single crystal. Next, after stopping the supply of the silane gas and the propane (4) and exhausting both of the gases, the substrate temperature was decreased to normal temperature at a rate of 50°C per minute.

The element concentrations of nitrogen, boron, aluminum, phosphorus, sulfur, titanium, vanadium, chromium, and molybdenum in the silicon carbide epitaxial film obtained through the above-described procedure were determined by secondary ion mass spectrometry (IMS 7f-Auto, manufactured by CAMECA). Table 7 shows the results.

[Table 7]

| | | Element Concentrations (atom/cm$^3$) | | | | | | | | |
|---|---|---|---|---|---|---|---|---|---|---|
| | | N | B | Al | P | S | Ti | V | Cr | Mo |
| Ex. 7 | Propane (4) | Less than 1 x 10$^{14}$ | Less than 1 x 10$^{15}$ | Less than 5 x 10$^{13}$ | Less than 3 x 10$^{14}$ | Less than 5 x 10$^{14}$ | Less than 4 x 10$^{14}$ | Less than 2 x 10$^{15}$ | Less than 6 x 10$^{14}$ | Less than 7 x 10$^{15}$ |
| Comp. Ex. 3 | Propane (1) | 6 x 10$^{14}$ | 3 x 10$^{15}$ | 7 x 10$^{14}$ | 7 x 10$^{14}$ | 9 x 10$^{14}$ | 9 x 10$^{14}$ | 1 x 10$^{16}$ | 3 x 10$^{15}$ | 1 x 10$^{16}$ |

[Comparative Example 3]

A silicon carbide epitaxial film was formed on the surface of the hexagonal silicon carbide single-crystal substrate using the same method as that of Example 7, except that the propane (1) was used instead of the propane (4). The element concentrations of nitrogen, boron, aluminum, phosphorus, sulfur, titanium, vanadium, chromium, and molybdenum in the silicon carbide epitaxial film were determined. Table 7 shows the results.

The following can be seen from the results of Example 7 and Comparative Example 3. That is, when the contents of the impurities in the propane were reduced, the element concentrations of nitrogen, boron, aluminum, phosphorus, sulfur, titanium, vanadium, chromium, and molybdenum in the silicon carbide epitaxial film were able to be reduced.

Reference Signs List

1 to 4: cylinder
10: separation membrane module
12: non-permeated gas outlet
13: permeated gas outlet
30: adsorption tower
40: gas filter
200: cylinder

**Claims**

1. A method for producing hydrocarbon, the method comprising:

   a hydrogen gas removal step of removing hydrogen gas from crude hydrocarbon containing hydrocarbon having 1 or more and 4 or less carbon atoms and the hydrogen gas to obtain low-hydrogen hydrocarbon in which a content of hydrogen gas is 100 ppm by volume or less; and
   an adsorption step of bringing the low-hydrogen hydrocarbon into contact with an adsorbent to obtain high-purity hydrocarbon in which a content of hydrogen gas is 80 ppm by volume or less, a total content of nitrogen gas and oxygen gas is 5 ppm by volume or less, and a total content of boron, aluminum, phosphorus, sulfur, titanium, vanadium, chromium, and molybdenum is 250 ppb by mass or less,
   wherein the adsorbent includes a crystal having pores with a pore diameter of more than 0.3 nm and 3.5 nm or less, and a crystal form of the crystal is not a mordenite type.

2. The method for producing hydrocarbon according to claim 1, wherein the hydrocarbon is at least one among methane, ethane, ethylene, propane, n-butane, and isobutane.

3. The method for producing hydrocarbon according to claim 1, wherein the hydrocarbon is at least one among propane and ethylene.

4. The method for producing hydrocarbon according to claim 1, wherein the adsorption step is a step of adsorbing at least one among the nitrogen gas and the oxygen gas on the adsorbent from the low-hydrogen hydrocarbon.

5. The method for producing hydrocarbon according to claim 1, wherein the adsorption step is a step of adsorbing at least one among boron, aluminum, phosphorus, sulfur, titanium, vanadium, chromium, and molybdenum on the adsorbent from the low-hydrogen hydrocarbon.

6. The method for producing hydrocarbon according to claim 1, wherein the hydrogen gas removal step is a step of removing the hydrogen gas from the crude hydrocarbon using a separation membrane.

7. The method for producing hydrocarbon according to claim 1, further comprising a filtration step of causing the crude hydrocarbon, the low-hydrogen hydrocarbon, or the high-purity hydrocarbon to pass through a filter medium to remove at least one among boron, aluminum, phosphorus, sulfur, titanium, vanadium, chromium, and molybdenum from the crude hydrocarbon, the low-hydrogen hydrocarbon, and the high-purity hydrocarbon,
   wherein the filter medium is at least one filter among a membrane filter, a sintered metal filter, and a filter including a plurality of metal halide pellets.

8. A method for producing silicon carbide in which silicon carbide is produced by using, as a raw material, high-purity hydrocarbon produced with the method for producing hydrocarbon according to any one of claims 1 to 7.

# FIG. 1

# FIG. 2

# FIG. 3

# FIG. 4

# FIG. 5

EXHAUST

## INTERNATIONAL SEARCH REPORT

| International application No. |
| --- |
| **PCT/JP2023/040727** |

**A.    CLASSIFICATION OF SUBJECT MATTER**

*C07C 7/12*(2006.01)i; *C07C 9/04*(2006.01)i; *C07C 9/06*(2006.01)i; *C07C 9/08*(2006.01)i; *C07C 9/10*(2006.01)i; *C07C 9/12*(2006.01)i; *C07C 11/04*(2006.01)i; *B01D 39/06*(2006.01)n; *B01D 39/16*(2006.01)n; *B01D 39/20*(2006.01)n; *B01D 53/04*(2006.01)n; *B01D 53/22*(2006.01)n

FI:    C07C7/12; C07C9/04; C07C9/06; C07C9/08; C07C9/10; C07C9/12; C07C11/04; B01D39/06; B01D39/16 C; B01D39/20 A; B01D53/04; B01D53/22

According to International Patent Classification (IPC) or to both national classification and IPC

**B.    FIELDS SEARCHED**

Minimum documentation searched (classification system followed by classification symbols)

C07C7/12; C07C9/04; C07C9/06; C07C9/08; C07C9/10; C07C9/12; C07C11/04; B01D39/06; B01D39/16; B01D39/20; B01D53/04; B01D53/22

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
| --- | --- | --- |
| Y | JP 62-153390 A (TOHO GAS CO LTD) 08 July 1987 (1987-07-08)<br>claims, fig. 1 | 1-8 |
| Y | JP 2019-156760 A (KAWASAKI HEAVY IND LTD) 19 September 2019 (2019-09-19)<br>claims, paragraphs [0030]-[0050], fig. 1, 2 | 1-8 |
| Y | JP 2013-159576 A (SUMITOMO SEIKA CHEM CO LTD) 19 August 2013 (2013-08-19)<br>claims, paragraphs [0077]-[0082] | 1-8 |
| Y | CN 206521407 U (SHENWU TECHNOLOGY GROUP CO., LTD.) 26 September 2017 (2017-09-26)<br>example 2 | 1-8 |
| Y | JP 2017-131856 A (FUJIFILM CORP) 03 August 2017 (2017-08-03)<br>paragraphs [0002]-[0003] | 1-8 |

☑ Further documents are listed in the continuation of Box C.      ☑ See patent family annex.

| | |
| --- | --- |
| *     Special categories of cited documents:<br>"A"   document defining the general state of the art which is not considered to be of particular relevance<br>"D"   document cited by the applicant in the international application<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
| --- | --- |
| **17 January 2024** | **30 January 2024** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

## INTERNATIONAL SEARCH REPORT

International application No.

**PCT/JP2023/040727**

### C. DOCUMENTS CONSIDERED TO BE RELEVANT

| Category* | Citation of document, with indication, where appropriate, of the relevant passages | Relevant to claim No. |
|---|---|---|
| Y | WO 2007/052497 A1 (NATIONAL UNIVERSITY OF CORPORATION HIROSHIMA UNIVERSITY) 10 May 2007 (2007-05-10) paragraphs [0002]-[0004] | 1-8 |
| Y | JP 2013-129606 A (SUMITOMO SEIKA CHEM CO LTD) 04 July 2013 (2013-07-04) paragraph [0008] | 1-8 |
| Y | JP 2012-106961 A (SUMITOMO SEIKA CHEM CO LTD) 07 June 2012 (2012-06-07) claims | 1-8 |

Form PCT/ISA/210 (second sheet) (July 2022)

## INTERNATIONAL SEARCH REPORT
### Information on patent family members

| International application No. |
| --- |
| **PCT/JP2023/040727** |

| Patent document cited in search report | | | Publication date (day/month/year) | Patent family member(s) | | | Publication date (day/month/year) |
| --- | --- | --- | --- | --- | --- | --- | --- |
| JP | 62-153390 | A | 08 July 1987 | (Family: none) | | | |
| JP | 2019-156760 | A | 19 September 2019 | WO | 2019/176744 | A1 | |
| | | | | DE | 112019001266 | T | |
| JP | 2013-159576 | A | 19 August 2013 | US | 2014/0378721 | A1 | |
| | | | | claims, paragraphs [0088]-[0093] | | | |
| | | | | WO | 2013/114667 | A1 | |
| | | | | TW | 201332966 | A | |
| | | | | KR | 10-2014-0128941 | A | |
| CN | 206521407 | U | 26 September 2017 | (Family: none) | | | |
| JP | 2017-131856 | A | 03 August 2017 | (Family: none) | | | |
| WO | 2007/052497 | A1 | 10 May 2007 | (Family: none) | | | |
| JP | 2013-129606 | A | 04 July 2013 | US | 2014/0343341 | A1 | |
| | | | | paragraphs [0009]-[0011] | | | |
| | | | | WO | 2013/094281 | A1 | |
| | | | | TW | 201332967 | A | |
| | | | | KR | 10-2014-0107246 | A | |
| JP | 2012-106961 | A | 07 June 2012 | (Family: none) | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5822299 B

- WO 2016121622 A